# EUROPEAN PATENT SPECIFICATION

(11) **EP 1 545 320 B1**
(45) Date of publication and mention of the grant of the patent: **23.06.2010**
(21) Application number: 03752618.3
(22) Date of filing: 25.09.2003
(51) Int. Cl.: A61B 17/00

(54) **MULTI-PURPOSE SURGICAL INSTRUMENT**
CHIRURGISCHES MULTIFUNKTIONSINSTRUMENT
INSTRUMENT CHIRURGICAL MULTI-USAGE

(30) Priority: 04.10.2002 US 264555; 02.05.2003 US 428706
(43) Date of publication of application: 29.06.2005
(73) Proprietor: Tyco Healthcare Group LP, Norwalk, CT 06850 (US)
(72) Inventor: BAYER, Hanspeter, Robert, Meriden, CT 06451-2840 (US); HEINRICH, Russell, Madison, CT 06443 (US)
(74) Representative: HOFFMANN EITLE
(86) International application number: PCT/US2003/030396
(87) International publication number: WO 2004/032753

(56) References cited:
- US-A- 5 431 672
- US-A1- 2002 098 138

## Description

### BACKGROUND

### Technical Field

The present invention relates to the field of surgical tools and more particularly to a multipurpose surgical tool for use during hand-assisted laparoscopic surgery, minimally invasive surgical procedures and traditional open surgical procedures.

### Background of Related Art

Open surgery, in general, has remained the procedure of choice for many surgeons since it enhances a surgeon's view of the operating cavity and allows a surgeon to readily palpate the internal organs as needed during a surgical procedure. However, the relatively large incisions required can often be traumatic for patients and may result in a prolonged healing process. As a result and as an alternative to traditional open surgery, many surgeons utilize minimally invasive surgical techniques to treat tissue remotely through small incisions utilizing specialized endoscopic instruments. More particularly, endoscopic instruments are inserted into the patient through a cannula or port that has been made with a trocar. Typical sizes for cannulas range from three millimeters to twelve millimeters. Smaller cannulas are usually preferred, which, as can be appreciated, ultimately presents a design challenge to instrument manufacturers who must find ways to make surgical instruments that fit through the smaller cannulas.

Certain endoscopic surgical procedures require cutting, cauterizing and/or sealing blood vessels and/or vascular tissue which, typically, requires the surgeon to insert different instruments through the working lumen of the endoscope to treat the tissue. As can be appreciated, this simply adds to the overall complexity of the operation since it requires the repeated exchange of surgical instruments through the working lumen to perform the different tasks associated with the particular surgery involved.

There are also some disadvantages to endoscopic surgery. For example, endoscopic instruments tend to limit a surgeon's ability to freely manipulate organs and often limit the surgeon's view of the operating cavity. Moreover, when using endoscopic instruments, the surgeon loses tactile feedback of the tissue which can play an important role in some surgical procedures. Further, when the particular surgery dictates the removal of a tissue specimen, the tissue must be either be morselized to fit through the trocar lumen or the surgeon must create a larger opening to remove the specimen intact essentially abandoning the benefits associated with endoscopic surgery.

Combining the advantages of the traditional and the laparoscopic techniques for abdominal surgery is commonly referred to as "hand-assisted laparoscopic surgery" (HALS). In this procedure, the normal laparoscopic small puncture openings are made with the exception that one opening is made late in the procedure and large enough to allow a surgeon's hand to pass through the opening to manipulate tissue, deliver new instruments into the operating cavity and/or remove tissue specimens. HALS attempts to restore dexterity and tactile feedback by allowing the surgeon to place one hand within the operating space through a hand access sleeve. Once the hand is in the operating space, it can be used to manipulate and palpate tissues in much the same way as it is used in open surgical procedures.

When performing surgery in the abdominal cavity, air or gas is typically introduced to create a condition known as "pneumoperitoneum". Ideally, HALS procedures are performed while maintaining the pneumoperitoneum which eliminates re-insufflation of the surgical cavity. As can be appreciated, if a surgeon's hand has to be removed to retrieve additional surgical instruments, the cavity will deflate and subsequent re-insufflation of the pneumoperitoneum may be required. This simply prolongs the overall surgical procedure and makes HALS surgery very tedious especially when multiple instruments must be utilized during the surgical procedure. As a result, a need exists to develop an improved surgical tool which reduces the need to remove the instruments useful for performing hand assisted laparoscopic surgical procedures.

U.S. Patent Application Publication US 2002/0098138 A1 discloses a sterile container for the accommodation and sterile storage of surgical instruments or material. The container comprises a plurality of separate chambers each having its own closure element.

### SUMMARY

The present disclosure relates to a multipurpose surgical tool for use in minimally invasive surgical procedures such as Hand Assisted Laparoscopic Surgery (HALS). Additionally, the multipurpose surgical tool of the present disclosure may be used in traditional open surgical procedures.

During HALS surgery, for example the multipurpose surgical tool may be inserted, in the hand, through a hand access sleeve which is made during the course of the surgery. The surgeon manipulates the tool as needed and switches among the plurality of specialized surgical instruments encased within the tool. As can be appreciated, this eliminates the need for the surgeon to remove his/her hand from the operating cavity to switch among instruments.

The present invention relates to a multi-instrument surgical tool which includes a housing having a plurality of ports disposed therein. The housing includes a corresponding plurality of elongated channels each in communication with a respective one of the plurality of ports. A plurality of surgical instruments are mounted in the housing at least one surgical instrument is mounted in one of the ports for selective deployment from the housing. At least one of the instruments has a slide member attached to the instrument and accessible from an exterior of the housing.

In one preferred embodiment, the housing includes a slot in communication with the elongated channel for receiving the slide member therethrough. The slot and the elongated channel may be open on an end of the housing and the slide member and the instrument may be slidable in a distal direction in the channel by manipulation of the slide member so as to deploy the instrument from the channel.

The instruments may comprise any combination of the following: vessel sealing devices; dissectors; resectors; probes; morselators; ultrasonic instruments; video-assisted devices; clip appliers; surgical staplers; coagulators; irrigation instruments; and suction instruments. The foregoing is merely by way of an example and other instruments may be included.

In certain preferred embodiments, the elongated channels are configured for interchanging different surgical instruments. The elongated channels may include a first set of channels dimensioned to accommodate a first category of surgical instruments and a second set of channels dimensioned to accommodate a second set of instruments.

The multi-instrument surgical tool may include a surgical scalpel. In a preferred embodiment, the surgical scalpel includes a proximal end dimensioned to slideably engage one of the ports of the housing. The surgical scalpel desirably includes a distal end having a cutting edge. The tool includes a safety guard which is movable relative to the scalpel from a first position wherein the guard substantially covers the cutting edge of the scalpel to a second position wherein the cutting edge is exposed.

The guard desirably includes a locking tab for releasably locking the guard in the second position. The guard may be spring-biased to return to the first position.

In one preferred embodiment, the housing includes a slot in communication with the elongated channel for receiving the slide member therethrough. The slot and the elongated channel may be open on an end of the housing and the slide member and the instrument may be slidable in a distal direction in the channel by manipulation of the slide member so as to deploy the instrument from the channel.

The instruments may comprise any combination of the following: vessel sealing devices; dissectors; resectors; probes; morselators; ultrasonic instruments; video-assisted devices; clip appliers; surgical staplers; coagulators; irrigation instruments; and suction instruments. The foregoing is merely by way of an example and other instruments may be included.

In certain preferred embodiments, the elongated channels are configured for interchanging different surgical instruments. The elongated channels may include a first set of channels dimensioned to accommodate a first category of surgical instruments and a second set of channels dimensioned to accommodate a second set of instruments.

### BRIEF DESCRIPTION OF THE DRAWINGS

Other objects and features of the present invention will become apparent from the following detailed description considered in connection with the accompanied drawings. It should be understood, however, that the drawings are designed for the purpose of illustration only and not as a definition of the limits of the invention.

Illustrative embodiments of the subject surgical tool are described herein with reference to the drawings, wherein only the tools shown in Figs. 1-2B, 3A-3E, and 4A-4C are embodiments of the present invention, whereas the tools shown in Figs. 2C, 2D, and 3F do not fall under the scope of the appended claims:

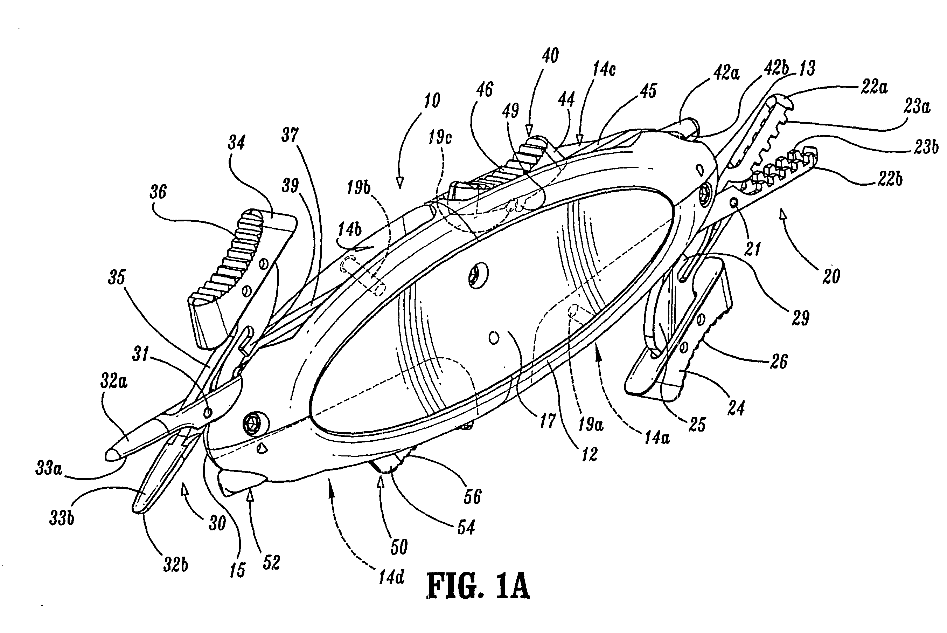

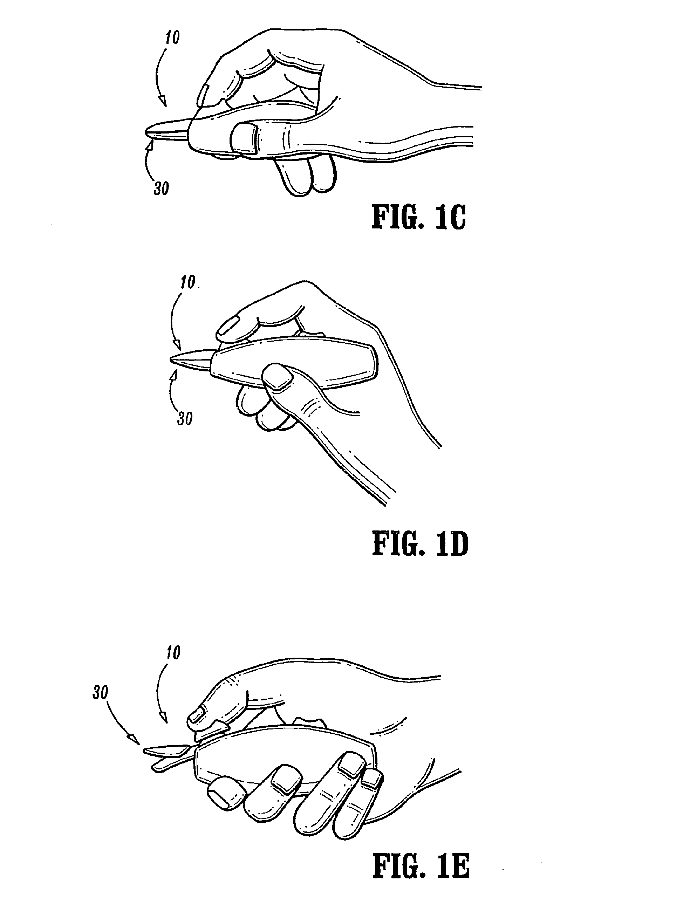

Fig. 1A is a perspective view of multi-instrument surgical tool in accordance with the present disclosure;

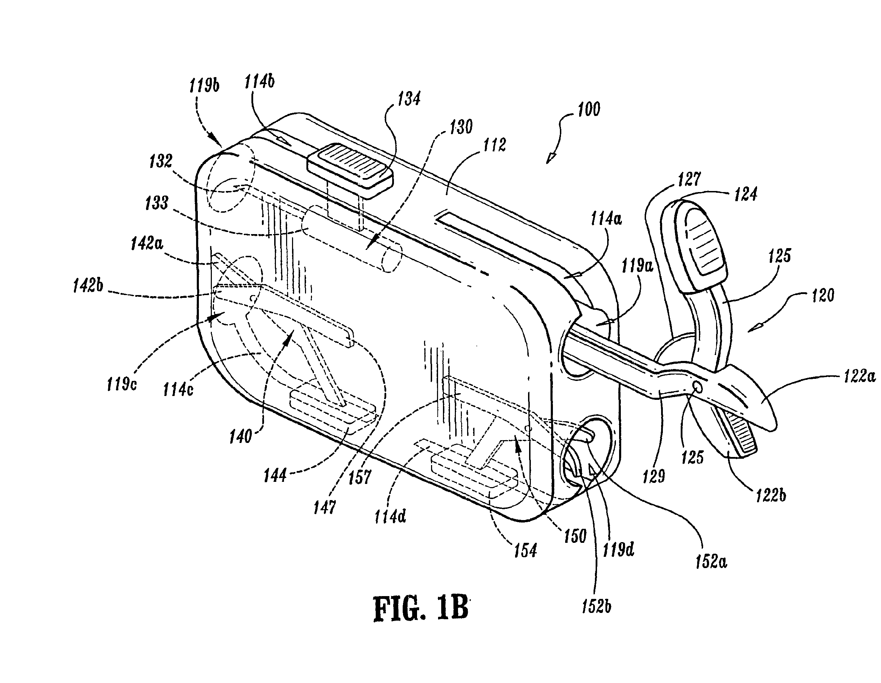

Fig. 1B is a perspective view of a multi-instrument surgical tool in accordance with an embodiment of the present invention;
Figs. 1C-1E are schematic representations showing various hand orientations for using an instrument according to the embodiment of Fig. 1B;

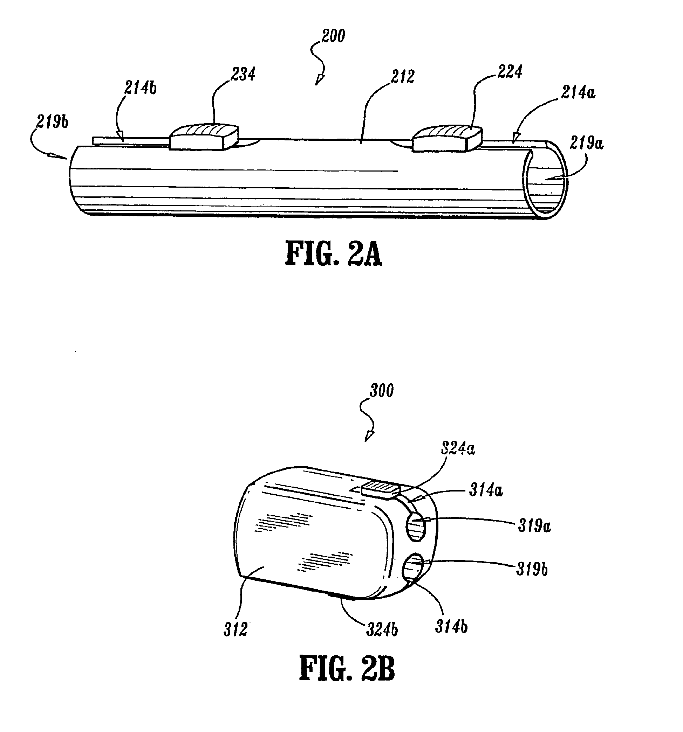

Fig. 2A is a side perspective view showing a multi-instrument surgical tool according to a further embodiment of the present invention;
Fig. 2B is a side perspective view showing a multi-instrument surgical tool according to another embodiment of the present invention;

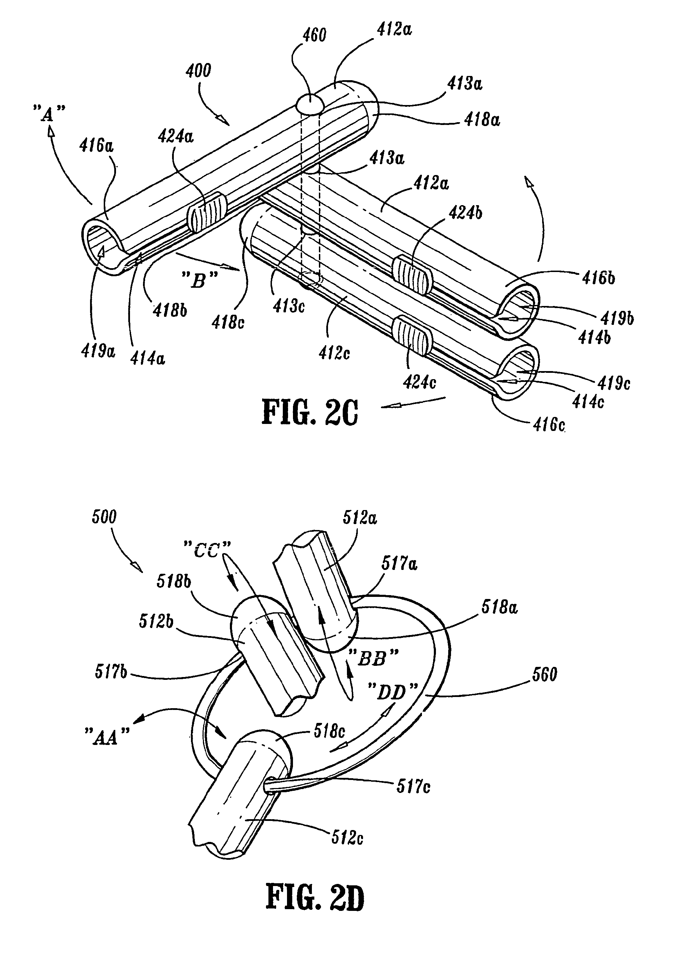

Fig. 2C is a perspective view showing a multi-instrument surgical tool according to another example;
Fig. 2D is a perspective view showing a multi-instrument surgical tool according to another example;

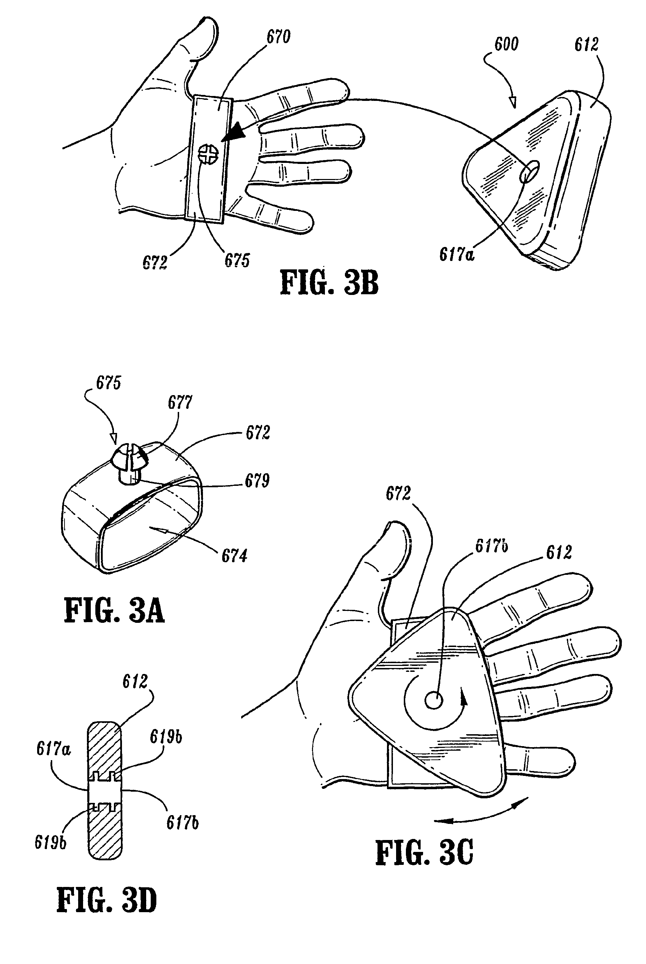

Fig. 3A is a perspective view of a mounting strap for use with a surgical tool in a further embodiment of the present invention;
Fig. 3B is a schematic view showing the proper orientation of the mounting strap of Fig. 3A;
Fig. 3C is a schematic view showing a surgical tool in the embodiment of Figs. 3A - 3B;
Fig. 3D is a cross section of the mounting strap in accordance with the embodiment of Figs. 3A -3C;

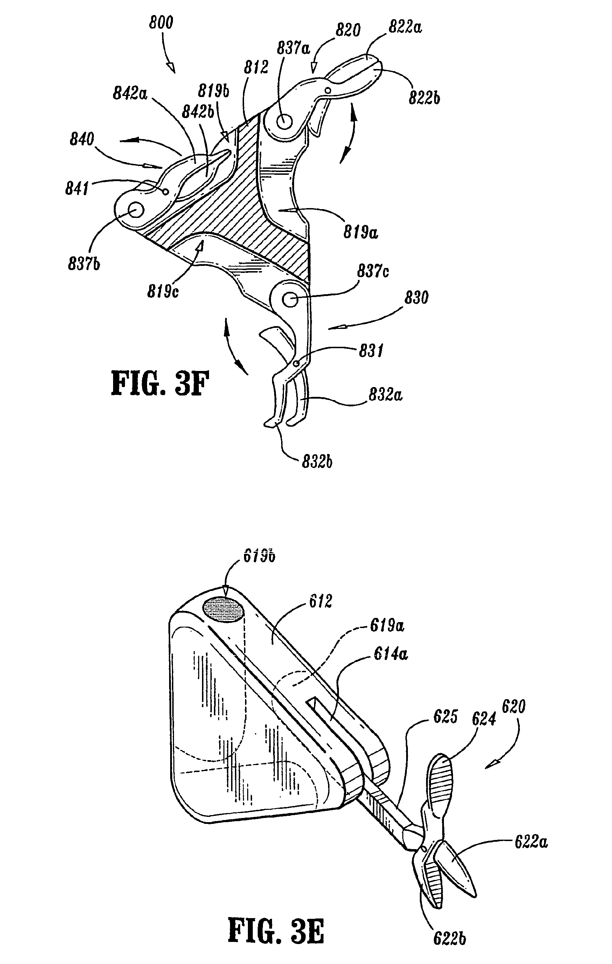

Fig. 3E is a perspective view of a multi-instrument surgical tool in accordance with a further embodiment of the invention;
Fig. 3F is a cross-sectional view of a multi-instrument surgical tool according to another example;

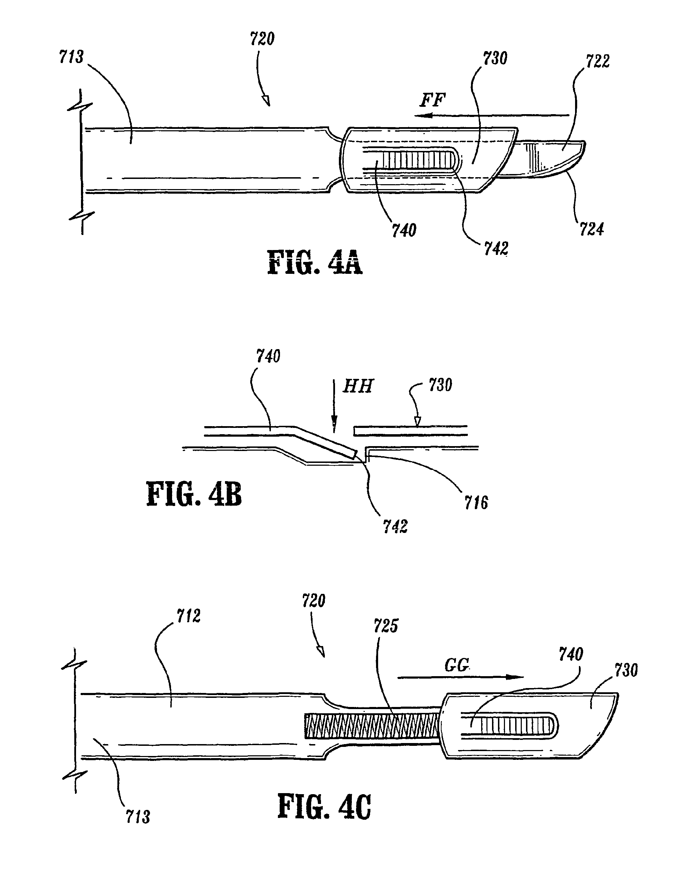

Fig. 4A is a side view of a multi-instnunent surgical tool in accordance with another embodiment of the present invention;
Fig. 4B is a schematic illustration of a locking mechanism for the embodiment of Fig. 4A; and
Fig. 4C is a side view of the multi-instrument surgical tool in accordance with the embodiment of Figs. 4A - 4B.

### DETAILED DESCRIPTION

Preferred embodiments of the multipurpose surgical tool will be described in terms of a HALS procedure wherein the typical laparoscopic incisions are made with the exception that one, larger opening is made during the course of the procedure which is large enough to allow a surgeon's hand to pass through the opening to manipulate tissue, deliver new instruments into the operating cavity and/or remove tissue specimens. Air or gas is introduced to create the pneumoperitoneum and the HALS procedure is performed while maintaining the pneumoperitoneum.

In the drawings and in the description which follows, the term "proximal", as is traditional, will refer to the end of the instrument which is closer to the user, while the term "distal" will refer to the end which is further from the user. When described in accordance with a particular figure, the proximal and distal ends are designated for the purpose of clarification only since the nature and use of the surgical tool of the present invention enables the surgeon to freely position and rotate the tool within the surgeon's hand.

Referring now in detail to the drawing figures in which like reference numerals identify similar or identical elements, one embodiment of the present disclosure is illustrated generally in Fig. 1A and is designated therein as multipurpose surgical tool 10. Surgical tool 10 includes a case or housing 12 which has a plurality of elongated instrument slots or ports 14a, 14b, 14c and 14d defined therein for removably housing a corresponding plurality of surgical instruments 20, 30, 40 and 50, respectively. More particularly, Fig. 1A shows one embodiment of a multipurpose surgical tool 10 which includes four surgical instruments 20, 30, 40 and 50 mounted for selective deployment within elongated ports 14a, 14b, 14c and 14d. In further embodiments, two or more instruments are provided on the tool.

Turning back to the embodiment shown in Fig. 1A, each of the corresponding plurality of surgical instruments, e.g., 20, is deployably housed within a corresponding elongated port 14a defined within the housing 12. With the four-instrument embodiment, two elongated ports 14a and 14c are located at one end, e.g., proximal end 13, for housing instruments 20 and 40, respectively, and two elongated ports, e.g., 14b and 14d are located at the opposite end, e.g., distal end 15, for housing instruments 30 and 50. As can be appreciated, each elongated port, e.g., 14a, is dimensioned to house the particular port's surgical instrument 20 and, as such, the relative dimensions of each port 14a may vary depending upon the particular instrument 20 being housed. Preferably, the outer surface of the housing 12 includes an ergonomically enhanced scallop to facilitate handling the tool 10 under wet operating conditions.

Alternatively, it is envisioned that all or some of the elongated ports 14a-14d may be dimensioned in a substantially uniform manner to enable an interchange of different surgical instruments 20, 30, 40 and 50. For example, it is envisioned that the surgeon may be able to select a combination of surgical instruments needed for a particular operation and assemble the tool 10 with these selected instruments prior to insertion. Preferably, the surgical instruments are selected from a group consisting of: needle holders; needle drivers; graspers; forceps; vessel sealing devices; dissectors; resectors; probes; morselators; ultrasonic instruments; video-assisted devices; clip appliers; surgical staplers; coagulators; bipolar an mechanical scissors; irrigation instruments; and suction instruments.

Moreover, it is also envisioned that certain elongated ports, e.g., 14a and 14c, may be dimensioned to accommodate one category of surgical instruments, e.g., 20 and 40, and the remaining ports 14b and 14d may be dimensioned to accommodate a second category of surgical instruments, e.g., 30 and 50. The surgeon is then able to interchange among the different categories of surgical instruments and removably engage the selected instruments in the correspondingly dimensioned or appropriate elongated ports as needed.

Fig. 1A shows the multi-purpose surgical instrument 10 having a forceps 30 deployed at the distal end 15 of the housing 12. The forceps includes two opposing jaw members 32a and 32b which are movable relative to one another about pivot 31 by virtue of an actuator 34. The jaw members 32a and 32b have inner facing surfaces 33a and 33b which cooperate to grasp tissue therebetween upon selective movement of the actuator 34. Preferably, the actuator 34 includes an ergonomically enhanced thumb tab 36 to facilitate deployment of the forceps 30 and subsequent actuation of the jaw members 32a and 32b within the operating cavity under wet conditions. A lever arm 35 is disposed between the actuator 34 and the movable jaw member, e.g., 32b, to increase the grasping pressure by mechanical advantage.

The forceps 30 also includes a locking flange 39 which engages a corresponding locking post 19b to releasably lock the forceps 30 within the corresponding elongated port 14b when retracted within housing 12. Preferably, a spring arm or leaf spring 37 biases the locking flange 39 against the locking post 19b in a pre-loaded configuration. As can be appreciated, when the actuator 34 is retracted proximally, the actuator 34 releases the locking flange 39 from the locking post 19b and allows the forceps 30 to deploy from the housing 12, i.e., the pre-loaded spring arm 37 forces the forceps 30 distally or outwardly from the housing 12 when released. Once deployed from elongated port 14b, the spring arm 37 also biases the jaws 32a and 32b in an open configuration to facilitate approximation of the tissue. Downward movement of the actuator 34 moves the jaw members 32a and 32b relative to one another about pivot 31 to grasp tissue.

A second instrument, namely, a grasping forceps 20, is shown deployed from the proximal end of the housing 12 of tool 10. Grasping forceps 20 includes two opposing jaw members 22a and 22b which are movable relative to one another about pivot 21 in much the same manner as forceps 30, and may also include an actuator 24. Preferably, the inner facing surfaces 23a and 23b of the jaw members 22a and 22b are corrugated to facilitate grasping and manipulation of tissue.

Grasping forceps 20 includes many of the same or similar components as forceps 30. For example, grasping forceps 20 also deploys in much the same fashion as forceps 30. An ergonomically enhanced tab surface 24 may be included for disengaging the locking flange 29 from locking post 19a to release spring arm (not shown). The forceps 20 also releasably locks into housing in a similar manner and a locking flange 29 and a corresponding locking post 19a is desirably provided fro locking into the housing. As can be appreciated, both of the forceps 20 or 30 may be deployed at any one time or the locking posts 19a and 19b may be interconnected (either mechanically or electro-mechanically) to allow deployment of only one instrument at a time.

Fig. 1A also shows third and fourth instruments 40 and 50 which are shown in a retracted or "housed" position within housing 12. These instruments 40 and 50 may include any of the instruments identified in the group described above. Preferably, each of these instruments is designed to lock within the housing 12 and deploy from the housing 12 in much the same fashion and described above with respect to forceps 20 and 30. Moreover, each of these instruments desirably includes similar components as described above which have similar functions. For example, instrument 40 includes an actuator 44, ergonomic surface 46, lever arm 45, jaw members 42a and 42b and locking flange 49 (which engages locking post 19c). Instrument 50 includes an actuator 54, ergonomic surface 56 and jaw members 52 which again cooperate in much the same fashion as described above.

Fig. 1B shows another embodiment of the surgical tool 100 of the present disclosure which shows a plurality of surgical instruments (forceps 120, needle driver 130, shears 140 and curved shears 150) for use with a housing 112. The housing may have a rectilinear shape, or any other shape. Each instrument 120, 130, 140 and 150 is housed within a respective port 119a, 119b, 119c and 119d. Each instrument, e.g., forceps 120, is manually deployable. Desirably, each instrument has a corresponding slide-like actuator or slide member, e.g., 124, which is movable distally (or forwardly) within an elongated slot 114a disposed within the corresponding port 119a. One arm 125 of the forceps 120 is attached at one end to the slide member 124 and at the other end to a corresponding jaw member 122b. The other jaw member 122a is attached to a second arm 129 which slidingly reciprocates within port 119a. Initial distal movement of the slide member 124 rides arm 125 along slot 114a until the slide member 124 releases from slot 114a. Preferably, a spring 127 biases the slide member 124 against arm 129 which, in turn, biases the jaw members 122a and 112b in an open configuration for approximating tissue.

To retract or store the forceps 120 in the housing 112, the surgeon simply re-engages the slide member 124 within slot 114a and moves the slide member 124 proximally along slot 114a until substantially all of the forceps 120 is seated within port 119a. Preferably, the spring 127 holds the forceps 120 in friction-fit engagement within slot 114a. Alternatively, a locking mechanism (not shown) may be employed to secure the forceps 120 within port 119a.

The other instruments, namely, needle driver 130, shears 140 and curved shears 150, all include similar components and are deployable in a similar fashion. More particularly, needle driver 130 includes a slide member 134 which slides along slot 114b to selectively deploy a needle tip 132 through port 119b. Shears 140 include a slide member 144 which slides along slot 114c to selectively deploy arm 147 and blade members 142a and 142b through port 119c. Curved shears 150 include a slide member 154 which slides along slot 114d to selectively deploy arm 157 and blade members 152a and 152b through port 119d.

As best illustrated in Figs. 1C - 1E, the surgeon can grasp and hold the surgical tool 10, 100 in a variety of different orientations for utilizing the different instruments contained therein. Moreover, the symmetrical aspects of the surgical tool 10, 100 allow the surgeon to comfortably use the tool 10, 100 in either a right-handed or left-handed orientation.

Fig. 2A shows an alternative embodiment of a two-instrument surgical tool 200 which utilizes ports disposed on opposite sides of an elongated housing 212. In the embodiment shown, two-instruments are provided, each in a corresponding port. The housing may have a tube-like shape, or any other shape. Each of the housed instruments (not shown) desirably includes a slide-like actuator or slide member 224 and 234 which moves along a corresponding slot 214a and 214b disposed within the housing 212 to deploy the particular instrument as needed during a given surgical procedure much in the same (or similar) manner as described above with respect to the embodiment of Figs. 1A-1D.

Fig. 2B shows another version of a surgical tool 300 which utilizes two ports 319a and 319b disposed adjacent one another (i.e., on the same side). Each instrument (not shown) includes an actuator 324a and 324b which moves along a corresponding slot 314a and 314b disposed within the housing 312 to deploy the particular instrument in the same (or similar) manner as described above.

Fig. 2C shows an alternative example of a surgical tool 400 which utilizes three instrument housings 412a, 412b and 412c. Each instrument housing has a respective instrument port 419a, 419b and 419c disposed at a distal end 416a, 416b and 416c of each housing (412a, 412b and 412c). The instrument housings 412a, 412b and 412c are commonly attached to an elongated rod or cylinder 460 which engages each housing 412a, 412b and 412c through an aperture 413a, 413b and 413c located at a proximal end 418a, 418b and 418c of housings 412a, 412b and 412c, respectively. Each instrument (not shown) desirably includes an actuator 424a, 424b and 424c which moves along a corresponding slot 414a, 414b and 414c disposed within housings 412a, 412b and 412c to deploy the particular instrument in the same (or similar) fashion as described with respect to Figs. 1A-1E.

As can be appreciated, the user simply rotates one of the three housings, e.g., 412a, which contains a particular surgical instrument (not shown) about rod 460 in the direction "A" and slides the actuator 424a distally to deploy the surgical instrument for introduction into the surgical field. To select a different instrument, the user retracts slide 424a, rotates housing 412a back into vertical registration with the other housings 412b and 412c in the direction "B" and rotates a new housing, e.g., 412b, in the direction "A" to selectively deploy another instrument (not shown).

Fig. 2D shows another version of a surgical tool 500. Tool 500 has housings 512a, 512b and 512c commonly mounted to a ring 560 through an aperture 517a, 517b and 517c located at the proximal end 518a, 518b and 518c of housings 512a, 512b and 512c, respectively. Much like the embodiment of Fig. 2C, the user simply rotates one of the three housings, e.g., 512a, about ring 560 in the direction of the arrows "AA", "BB" and "CC" to position the surgical instrument for introduction into the surgical field. The user can also move a particular instrument in the direction "DD" along ring 560 to facilitate selection and handling of the instrument in the surgical field.

Figs. 3A-3D show another embodiment of a surgical tool 600 which utilizes a mounting strap or hand clip 670 to permit selective rotation of the surgical tool within a surgeon's hand. Preferably, hand clip 670 includes at least one mechanical interface which mates with a corresponding mechanical interface disposed on the housing 612 of the surgical tool 600. More particularly and as shown in Figs. 3A and 3B, hand clip 670 includes a press-lock fitting 675 which mechanically engages a corresponding aperture 617a (or 617b) located within the outer periphery of the housing 612. As best shown in Figs. 3A and 3D, the press lock is designed for snap-fit engagement within aperture 617a (or 617b).

The press-lock shown includes a segmented top portion 677 which is supported by a stem section 679. Upon introduction of the top portion 677 into one of the two apertures 617a or 617b (for right-handed or left-handed use of the surgical tool, respectively), the segmented top portion 677 initially compresses inwardly to facilitate engagement within the respective aperture 617a (or 617b). Once the top portion 677 is fully engaged within aperture 617a (or 617b), the top portion 677 expands or "snaps" into engagement with a ring-like flange 619a (or 619b) disposed within the inner periphery of aperture 617a (or 617B). Preferably, the top portion 677 and the ring-like flange 619a (or 619b) are dimensioned to facilitate rotation of the surgical tool 600 relative to the hand clip 670 to allow a surgeon to select and orient the particular surgical tool 600 as needed during surgery (See Fig. 3C).

Fig. 3E shows the surgical tool 600 of Fig. 3B with a surgical grasping instrument 620 shown deployed from port 619a of housing 612 and ready for use. Much in the same (or similar) manner as described above with respect to the embodiment of Figs. 1A-1D, the grasping instrument 620 includes a slide-like actuator 624 which moves along slot 614a to extend the shaft 625 and jaws 622a and 622b from the housing 612 to enable use of the grasping instrument 620 as needed during a surgical procedure.

Fig. 3F shows another example of the surgical tool 800 according to the present disclosure wherein a plurality of surgical instruments 820, 830 and 840 are each housed in a corresponding recess 819a, 819b and 819c, respectively, disposed along the outer periphery of the housing 812. More particularly, each instrument, e.g., grasper 820, is rotatingly mounted to housing 812 about a pivot 837a and is selectively movable from a first "stored" position to a second "deployed" position. Preferably, each recess, e.g., 819a, is dimensioned to store the respective instrument, e.g., grasper 820, in a generally flush manner with respect to the outer periphery of the housing 812. Moreover, each recess 819A is preferably dimensioned such that the jaw members or end effectors 822a and 822b remain properly seated in a generally closed configuration when stored.

As can be appreciated, the other instruments of surgical tool 800 are deployed in much the same fashion as grasper 820 and include similar elements to those instruments described above. For example, instrument 830 is a curved forceps and includes jaws 832a and 832b which are rotatable about pivot 831 to approximate and grasp tissue. The forceps 830 mounts to housing 812 about pivot 837c and is stored within recess 819c when not is use. Likewise, scissors 840 include jaws 842a and 842b which are rotatable about pivot 841 to sever tissue. The scissors 840 mount to housing 812 about pivot 837b and are stored within recess 819b when not is use.

As shown best in Figs. 4A - 4C, one of the plurality of instruments may be a surgical scalpel 720 having proximal and distal ends 713 and 722, respectively. The proximal end 713 is preferably dimensioned to slideably engage one of the ports (not shown) of the housing (not shown) and the distal end 722 includes a cutting edge 724. A spring-loaded safety guard 730 is also included which is movable relative to the scalpel 720 from a first position wherein the guard 730 substantially covers the cutting edge 724 of the scalpel 720 (See Fig. 4C) to a second position wherein the cutting edge 724 is exposed.

Preferably, the safety guard 730 includes a locking tab 740 for releasably locking the guard 730 in the second position (See Fig. 4B). The guard 730 may also include a spring 725 which automatically extends the guard 730 once the locking tab 740 is released to cover the cutting edge 724 when not in use. More particularly, during use the user retracts the guard 730 proximally against spring 725 in the direction "FF" and simultaneously depresses locking flange 740 inwardly (i.e., in the direction of arrow "HH") such that a distal end 742 of the locking tab 740 abuts against a corresponding flange or notch 716 disposed within the outer periphery of scalpel 730 (See Fig. 4B). The biasing force of the spring 725 retains the locking tab 740 within the notch 716 and locks the guard 730 in a retracted position to expose the cutting edge 724 of the scalpel 720 for use. Once the surgeon has completed the cut using the scalpel 720, the surgeon simply retracts the guard 730 proximally (i.e., in the direction "FF") which releases the locking tab 742 from the notch 716 and allows the guard 730 to extend distally in the direction "GG" over the cutting edge 724 under the force of the spring 725.

## Claims

1. A multi-instrument surgical tool, comprising:
a) a housing having a plurality of ports disposed therein, the housing including a corresponding plurality of elongated channels each in communication with a respective one of the plurality of ports;
b) a plurality of surgical instruments mounted in the housing, at least one of the surgical instruments being mounted in one of the plurality of ports for selective deployment from the housing; and
c) at least one of the instruments having a slide member attached to the instrument and accessible from an exterior of the housing.

2. A tool according to claim 1 wherein one of the instruments includes a surgical scalpel which includes:
proximal and distal ends, the proximal end being dimensioned to slideably engage one of the ports of the housing and the distal end having a cutting edge;
a guard which is movable relative to the scalpel from a first position wherein the guard substantially covers the cutting edge of the scalpel to a second position wherein the cutting edge is exposed.

3. A tool according to claim 2 wherein the guard includes a locking tab for releasably locking the guard in the second position.

4. A tool according to claims 2 or 3 wherein the guard is spring-biased to return to the first position.

5. A tool according to any preceding claim wherein the housing includes a slot in communication with the elongated channel for receiving the slide member therethrough.

6. A tool according to any preceding claim wherein the elongated channel is open on an end of the housing and the slide member of the instrument is slideable in a distal direction in the channel by manipulation of the slide member so as to deploy the instrument from the channel.

7. A tool according to any preceding claim wherein the instruments are selected from the group consisting of vessel sealing devices, dissectors, resectors, probes, moselators, ultrasonic instruments, video-assisted devices, clip appliers, surgical staplers, coagulators, irrigation instruments and suction instruments.

8. A tool according to any preceding claim wherein the elongated channels include a first set of channels dimensioned to accommodate a first category of surgical instruments and a second set of channels dimensioned to accommodate a second category of surgical instruments.

9. A tool according to any preceding claim wherein a first instrument of the plurality of instruments comprises a locking flange and the housing includes a locking post disposed in one of the channels, the locking flange being arranged to engage the locking post when the first instrument is stored within the housing.

10. A tool according to any preceding claim wherein the housing includes at least one elongated channel in communication with the plurality of ports and wherein the plurality of ports includes a first port open at a first end of the housing and a second port open on a second end of the housing, the first end being opposite from the second end; and
each surgical instrument being slideably mounted in the at least one elongated channel for selective deployment from the housing.

## Patentansprüche

1. Chirurgisches Multi-Instrumentenwerkzeug, umfassend:
a) ein Gehäuse mit einer Mehrzahl von Anschlüssen, die darin angeordnet sind, wobei das Gehäuse eine entsprechende Mehrzahl länglicher Kanäle enthält, die jeweils in Verbindung mit einem entsprechenden der Mehrzahl von Anschlüssen stehen;
b) eine Mehrzahl chirurgischer Instrumente, die in dem Gehäuse montiert sind, wobei zumindest eines der chirurgischen Instrumente in einem der Mehrzahl von Anschlüssen zum wahlweisen Ausfahren aus dem Gehäuse montiert ist; und
c) zumindest eines der Instrumente mit einem Schiebeelement, das an dem Instrument angebracht und von einer Außenseite des Gehäuses zugänglich ist.

2. Werkzeug nach Anspruch 1, wobei eines der Instrumente ein chirurgisches Skalpell enthält, das enthält:
ein proximales und distales Ende, wobei das proximale Ende bemessen ist, um verschiebbar in einen der Anschlüsse des Gehäuses einzugreifen, und das distale Ende eine Schnittkante aufweist;
einen Schutz, der relativ zu dem Skalpell aus einer ersten Position, in der der Schutz im Wesentlichen die Schnittkante des Skalpells bedeckt, in eine zweite Position, in der die Schnittkante freigelegt ist, bewegbar ist.

3. Werkzeuganordnung nach Anspruch 2, wobei der Schutz eine Feststellnase zum lösbaren Feststellen des Schutzes in der zweiten Position enthält.

4. Werkzeug nach Anspruch 2 oder 3, wobei der Schutz federvorgespannt ist, um in die erste Position zurück zu gelangen.

5. Werkzeug nach einem vorhergehenden Anspruch, wobei das Gehäuse einen Schlitz in Verbindung mit dem länglichen Kanal zum Aufnehmen des Schiebeelements **dadurch** enthält.

6. Werkzeug nach einem vorhergehenden Anspruch, wobei der längliche Kanal an einem Ende des Gehäuses geöffnet ist, und das Schiebeelement des Instruments in einer distalen Richtung in dem Kanal durch Manipulation des Schiebeelements verschiebbar ist, um das Instrument aus dem Kanal auszufahren.

7. Werkzeug nach einem vorhergehenden Anspruch, wobei die Instrumente aus der Gruppe ausgewählt sind, die aus Gefäßabdichtvorrichtungen, Sezierern, Resektoren, Sonden, Moselatoren, Ultraschallinstrumenten, Videounterstützten Vorrichtungen, Klemmanbringern, chirurgischen Heftungen, Koagulatoren, Irrigationsinstrumenten und Sauginstrumenten besteht.

8. Werkzeug nach einem vorhergehenden Anspruch, wobei die länglichen Kanäle einen ersten Satz Kanäle, die bemessen sind, um eine erste Kategorie chirurgischer Instrumente aufzunehmen, und einen zweiten Satz Kanäle, die bemessen sind, um eine zweite Kategorie chirurgischer Instrumente aufzunehmen, enthalten.

9. Werkzeug nach einem vorhergehenden Anspruch, wobei ein erstes Instrument der Mehrzahl von Instrumenten einen Feststellflansch aufweist und das Gehäuse eine Feststellstütze enthält, die in einem der Kanäle angeordnet ist, wobei der Feststellflansch ausgestaltet ist, um mit der Feststellstütze einzugreifen, wenn das erste Instrument innerhalb des Gehäuses gelagert ist.

10. Werkzeug nach einem vorhergehenden Anspruch, wobei das Gehäuse zumindest einen länglichen Kanal in Verbindung mit der Mehrzahl von Anschlüssen enthält und wobei die Mehrzahl von Anschlüssen einen ersten Anschluss, der an einem ersten Ende des Gehäuses geöffnet ist, und einen zweiten Anschluss, der an einem zweiten Ende des Gehäuses geöffnet ist, enthält, wobei das erste Ende gegenüber dem zweiten Ende liegt; und
wobei jedes chirurgische Instrument verschiebbar in dem zumindest einen länglichen Kanal zum wahlweisen Ausfahren aus dem Gehäuse montiert ist.

## Revendications

1. Outil chirurgical multi-instrument, comprenant:
a) un boîtier dans lequel sont ménagés plusieurs orifices, le boîtier comportant une pluralité correspondante de canaux oblongs, chacun en communication avec un de la pluralité d'orifices;
b) plusieurs instruments chirurgicaux installés dans le boîtier, au moins un des instruments chirurgicaux étant installé dans un de la pluralité d'orifices pour le déploiement sélectif du boîtier; et
c) au moins un des instruments ayant un élément coulissant fixé à l'instrument et accessible depuis l'extérieur du boîtier.

2. Outil selon la revendication 1, dans lequel un des instruments comporte un scalpel chirurgical qui comporte:
des extrémités proximale et distale, l'extrémité proximale étant dimensionnée pour venir en prise de coulissement avec un des orifices du boîtier, et l'extrémité distale ayant un bord coupant;
une protection qui est déplaçable relativement au scalpel d'une première position dans laquelle la protection couvre sensiblement le bord coupant du scalpel, à une seconde position dans laquelle le bord coupant est exposé.

3. Outil selon la revendication 2, dans lequel la protection comporte une patte de verrouillage pour verrouiller relâchablement la protection dans la seconde position.

4. Outil selon les revendications 2 ou 3, dans lequel la protection est sollicitée par ressort à revenir dans la première position.

5. Outil selon l'une quelconque des revendications précédentes, dans lequel le boîtier comporte une fente en communication avec le canal oblong pour recevoir l'élément coulissant à travers celle-ci.

6. Outil selon l'une quelconque des revendications précédentes, dans lequel le canal oblong est ouvert sur une extrémité du boîtier, et l'élément coulissant de l'instrument peut coulisser dans une direction distale dans le canal en manipulant l'élément coulissant de manière à déployer l'instrument du canal.

7. Outil selon l'une quelconque des revendications précédentes, dans lequel les instruments sont sélectionnés dans le groupe consistant en dispositifs de scellement de vaisseaux, dissecteurs, résecteurs, sondes, mosélateurs, instruments ultrasoniques, dispositifs vidéo-assistés, applicateurs de pinces, agrafeuses chirurgicales, coagulateurs, instruments d'irrigation et instruments d'aspiration.

8. Outil selon l'une quelconque des revendications précédentes, dans lequel les canaux oblongs comportent un premier ensemble de canaux dimensionnés pour recevoir une première catégorie d'instruments chirurgicaux et un deuxième ensemble de canaux dimensionnés pour recevoir une deuxième catégorie d'instruments chirurgicaux.

9. Outil selon l'une quelconque des revendications précédentes, dans lequel un premier instrument de la pluralité d'instruments comprend une bride de verrouillage, et le boîtier comporte un montant de verrouillage disposé dans un des canaux, la bride de verrouillage étant agencée pour venir en prise avec le montant de verrouillage lorsque le premier instrument est stocké dans le boîtier.

10. Outil selon l'une quelconque des revendications précédentes, dans lequel le boîtier comporte au moins un canal oblong en communication avec la pluralité d'orifices, et où la pluralité d'orifices comprend un premier orifice ouvert à une première extrémité du boîtier et un deuxième orifice ouvert sur une seconde extrémité du boîtier, la première extrémité étant opposée à la seconde extrémité; et
chaque instrument chirurgical étant installé d'une manière coulissante dans le au moins un canal oblong pour le déploiement sélectif du boîtier.
